Europäisches Patentamt

(19) European Patent Office

Office européen des brevets

(11) Veröffentlichungsnummer: **0 026 527**
**B1**

(12) **EUROPÄISCHE PATENTSCHRIFT**

(45) Veröffentlichungstag der Patentschrift:
03.10.84

(51) Int. Cl.³: **C 12 J 1/10, C 12 M 1/36**

(21) Anmeldenummer: 80200876.3

(22) Anmeldetag: 18.09.80

(54) Verfahren und Anlage zur Herstellung von Gärungsessig.

(30) Priorität: 26.09.79 CH 8642/79

(43) Veröffentlichungstag der Anmeldung:
08.04.81 Patentblatt 81/14

(45) Bekanntmachung des Hinweises auf die Patenterteilung:
03.10.84 Patentblatt 84/40

(84) Benannte Vertragsstaaten:
AT BE CH DE FR GB IT LI NL

(56) Entgegenhaltungen:
CH - A - 375 315
FR - A - 1 214 936
FR - A - 1 418 296
FR - A - 2 021 600
FR - A - 2 374 415
FR - A - 2 374 416

Die Akte enthält technische Angaben, die nach dem
Eingang der Anmeldung eingereicht wurden und die
nicht in dieser Patentschrift enthalten sind.

(73) Patentinhaber: Process Engineering Company SA, Alte
Landstrasse 415, CH-8708 Männedorf (CH)

(72) Erfinder: Aeschbach, Hermann Albert, Ancien Stand 32,
CH-1820 Montreux (CH)

(74) Vertreter: Herrmann, Peter Johannes, c/o PPS
Polyvalent Patent Service AG Mellingerstrasse 1,
CH-5400 Baden (CH)

Anmerkung: Innerhalb von neun Monaten nach der Bekanntmachung des Hinweises auf die Erteilung des europäischen
Patents im Europäischen Patentblatt kann jedermann beim Europäischen Patentamt gegen das erteilte europäische Patent
Einspruch einlegen. Der Einspruch ist schriftlich einzureichen und zu begründen. Er gilt erst als eingelegt, wenn die
Einspruchsgebühr entrichtet worden ist (Art. 99(1) Europäisches Patentübereinkommen).

## Beschreibung

Gegenstand der Erfindung ist ein Verfahren und eine Anlage zur submersen, automatisch gesteuerten Herstellung von Gärungsessig aus Ethanol in zwei Stufen mit einer oder mehreren Vorstufen.

Aus der Schweizer Patentschrift 375 315 ist eine Vorrichtung zur Herstellung von Gärungsessigsäure bekannt, in der im wesentlichen mit 2 Gärbehältern gleicher Kapazität gearbeitet wird. Nach vollständiger Entleerung des einen Behälters, nachdem aller Alkohol verbraucht ist, wird dieser mit einem Teil aus dem zweiten Behälter beimpft und mit frischer Maische aufgefüllt. Der Versäuerungsendpunkt wird über das Absinken der Temperatur des Mediums bestimmt.

Ein Zweistufenverfahren ist auch nach der DE-PS 2 657 330 bekannt, welches zu Säurekonzentrationen über 15% Essigsäure führt, bei einer Alkoholkonzentration unter 0,5%. Danach wird in einem ersten Gärstadium die Bakterienvermehrung und Versäuerung vorgenommen und in einem zweiten Gärstadium die Versäuerung nach praktisch beendeter Vermehrung der Essigsäurebakterien.

Essigsäurebakterien, die sich nicht mehr vermehren, produzieren Essigsäure sehr langsam, was somit die Wirtschaftlichkeit des Verfahrens beeinflusst.

Weitere Nachteile der bekannten Verfahren sind, dass bei einer Steuerung des Essigausstosses über die Temperatur eine Messgrösse verwendet wird, die von äusseren Bedingungen beeinflusst werden kann und nicht durch den mikrobiologischen Prozess selbst. Ein weiterer Nachteil ist, dass bei einem erwähnten Verfahren die Verwertung des Alkohols in der Endvergärungsstufe unvollständig ist.

Aufgabe der Erfindung ist es, ein Verfahren zu schaffen, welches automatisch gesteuert quasi kontinuierlich unter vollständiger Verwertung des eingesetzten Alkohols zu Essigsäure führt.

Diese Aufgabe wird durch das Verfahren gemäss Anspruch 1 gelöst und ist dadurch gekennzeichnet, dass das Ernteventil und die Erntepumpe des Erntefermenters durch das Ansteigen des gelösten Sauerstoffes im Substrat nach praktisch vollständigem Verbrauch des Alkohols gesteuert werden können.

Während der Vergärung in einer Endstufe sinkt ohne Zufuhr vom neuen Substrat der Alkoholgehalt laufend ab, während bei konstanter Luftzufuhr der gelöste Sauerstoff bis zu dem Punkt konstant bleibt, bei welchem der Alkohol vollständig verbraucht ist. Als Steuerparameter zur Ermittlung des Endpunktes der Fermentation wird der Partialdruck ($pO_2$) des gelösten Sauerstoffs gemessen. Dieser kontrolliert den Verlauf der Fermentation, wobei die Messgrösse so umgeformt wird, dass bei normaler Produktivität ein Relativwert von 0% gelöstem Sauerstoff eingehalten wird. In dieser Phase erfolgt daher vollständige Absorption des eingebrachten Sauerstoffs. In der Endphase wird ein Relativwert von 100% gelöstem Sauerstoff erreicht, was den vollständigen Verbrauch des oxidierbaren alkoholischen Substrates bedeutet. Das Ansteigen der $pO_2$-Messgrösse wird mittels Grenzkontakten im Bereich von 70–100% zur Auslösung der Steuerung genutzt.

Die Versäuerung erfolgt bis zur Endkonzentration des biologisch oxidierbaren Ethanols, was praktisch Restalkohol Null bedeutet. Die Endstufe ohne Restalkohol wird vollständig entleert. Danach wird der leere Gärbehälter der Endstufe wieder automatisch mit einem Teilvolumen des Substrates zusammen mit den Essigsäurebakterien aus einem oder mehreren Vorfermentern befüllt, während dem Vorfermenter neue Maische zugeführt wird. Die Vorversäuerung erfolgt bis zu einem Alkoholgehalt von 0,5 bis 3 Volumenprozent. Das Volumenverhältnis von Vorfermenter zu Endfermenter beträgt 1,7 bis 3.

Die Erfindung soll beispielhaft anhand einer Zeichnung erläutert werden.

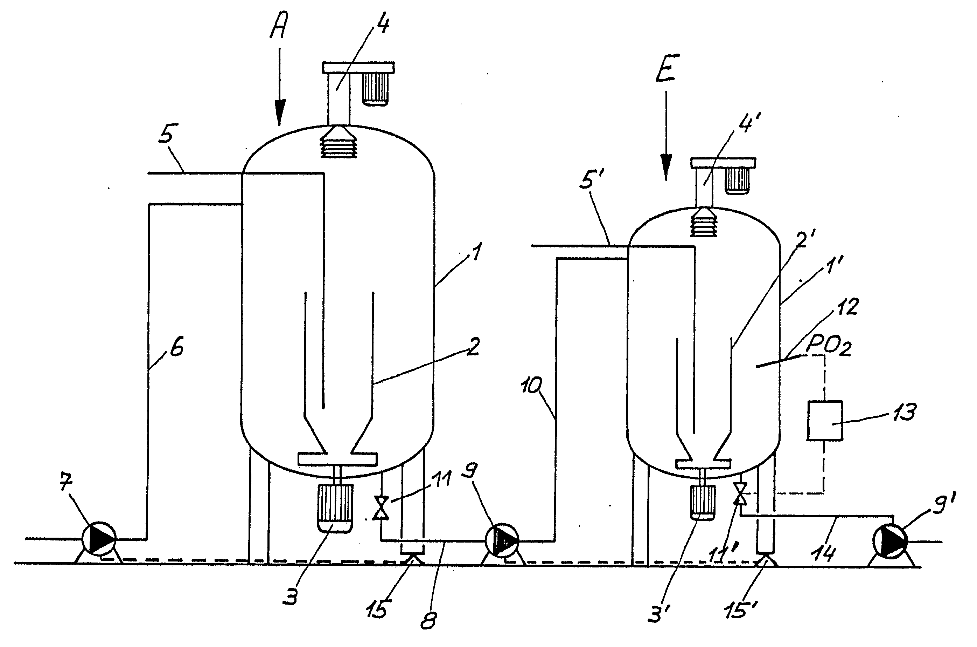

In der einzigen Figur besteht die Anlage im einfachsten Fall aus einem Vorfermenter A und einem Erntefermenter E. Der Vorfermenter A weist das 1,7 bis 3,5fache des Volumens vom Erntefermenter E auf. Beide Fermenter bestehen aus einem Behälter 1 bzw. 1', einem zentralen Leitrohr 2 bzw. 2', einem Antrieb 3 bzw. 3', einem mechanischen Schaumabscheider 4 bzw. 4'. Beide Behälter sind mit Hohlkörper-Strömungswehren zur Konstanthaltung der Temperatur sowie den Luftzufuhrsystemen 5 bzw. 5' versehen. Der Vorfermenter A weist eine Substratzuleitung 6 auf, die an eine Speisepumpe 7 angeschlossen ist. Eine Abgangsleitung 8 mit Ventil 11 führt vom Vorfermenter A über eine Pumpe 9 auf die Zugangsleitung 10 von Fermenter E. Der Fermenter E ist mit einem Ernteventil 11', einer Leitung 14 und einer Pumpe 9' ausgestattet. Das Ventil 11' wird von der Sauerstoffelektrode 12 über die Verstärkereinrichtung 13 gesteuert. Beide Fermenter sind mit Druckmessdosen 15 bzw. 15' zur Messung des Behälterinhaltes versehen.

Im Betrieb wird zunächst im Vorfermenter A bis zu einem Alkoholgehalt von 0,5 bis 3 Volumenprozent in bekannter Weise vorversäuert. Anschliessend wird ein Teilvolumen aus dem Vorfermenter A über das Leitungsstück 8 durch die Pumpe 9 und das Leitungsstück 10 in den Erntefermenter E gefördert, unter ständiger Belüftung mit einer Luftmenge von 6 m³ pro Stunde und m³ Arbeitsvolumen wie im Vorfermenter A belüftet und die eingestellte Temperatur konstantgehalten. Der Messwert des gelösten Sauerstoffgehaltes zeigt 0% an und geht in der Endphase auf 100%. Ab dem Zeitpunkt der neuen Beschickung des Endfermenters E wird mittels eines niveaugesteuerten Kontaktes eine wählbare temporäre Verriegelung des Ist-Sollwert-Vergleichs des $pO_2$-Wertes ausgelöst, welche verhindert, dass auftretende Einschwingungen bis zum konstanten $pO_2$-Verlauf nicht zur Auslösung der Erntesteuerung führen. In Serie der erwähnten Steuerungsbedingung muss über Niveaukontakte das erreichte Arbeitsvolumen des entsprechenden Vorfermenters als gegeben gemeldet werden, damit bei Störungen keine Endfermenterentleerung erfolgen kann, bevor der Vorfermenter seine Substratzufuhr beendet hat. Im Bereich von 70–100% öffnet sich das Ventil 11', wodurch der Inhalt des Fermenters E über die Leitung 14 der weiteren Aufarbeitung zugeführt wird.

Beim Vorgehen mit 2 Fermentern stabilisiert sich das biologische System bei kleinen Parameterschwankungen von selbst, mit Fermenten müssen bei Parameterschwankungen Korrekturen vorgenommen werden. An einem Berechnungsbeispiel soll das Gleichgewicht zwischen Teilerneuerung in der Vorstufe $V_A$ und der Endstufe $V_E$ = dargestellt werden:

$$V_A; \quad V_E \ [m^3]$$

$$(1) \quad V_A = \frac{V_E}{1-KQ} [m^3]$$

$$(2) \quad KQ = \frac{S_{NT}-S_S}{S_{VT}-S_S} [-]$$

$$(3) \quad S_{NT} = S_E - \Delta S \left[\frac{kg}{m^3}\right]$$

$$(4) \quad S_{VT} = S_E - \Delta S \frac{P_E}{P_A + P_E}$$

KQ  =  Konzentrationsquotient
$S_{NT}$  =  Essigsäure nach Teilerneuerung
$S_{VT}$  =  Essigsäure vor Teilerneuerung
$S_S$  =  Essigsäure im Substrat
$S_E$  =  Essigsäure in der Endstufe ohne Restalkohol
$\Delta S$  =  $S_E - S_{NT}$
$P_A, P_E$  =  Produktivität Endstufe bzw. Vorstufe [kg (Essigsäure 100%) · $h^{-1}$ · $m^{-3}$]

Die Vorteile dieses Verfahrens sind darin zu sehen, dass die Produktivität grösser ist als die Summe der Kapazitäten gleichdimensionierter einstufiger Fermenter, da im Chargenbetrieb der Erntestufe aktive Biomasse zugeführt wird. Durch den Chargenbetrieb der Erntestufe wird der durch die Acetobacter oxidierbare Alkohol vollständig verwertet.

## Patentansprüche

1. Verfahren zur submersen, automatisch gesteuerten Herstellung von Gärungsessig aus Ethanol in zwei Stufen, wobei Erntefermenter (E) für einen oder mehrere Vorfermenter (A) vorgesehen sind und die Vorfermenter (A) zur Erneuerung der Biomasse teilweise mit frischer Maische gefahren werden, dadurch gekennzeichnet, dass das Ernteventil (11') und die Erntepumpe (9') des Erntefermenters (E) durch das Ansteigen des gelösten Sauerstoffes im Substrat nach praktisch vollständigem Verbrauch des Alkohols gesteuert werden.

2. Verfahren nach Anspruch 1, dadurch gekennzeichnet, dass der Erntefermenter (E) nach Versäuern bis zu der durch die biologische Oxidation erreichbaren Endkonzentration an Ethanol durch Acetobacter vollständig entleert wird.

3. Verfahren nach den Ansprüchen 1 und 2, dadurch gekennzeichnet, dass nach beendetem Ausstoss der Endfermenter (E) automatisch aus dem Vorfermenter (A) aufgefüllt wird.

4. Verfahren nach den Ansprüchen 1 bis 3, dadurch gekennzeichnet, dass nach Auffüllung des Endfermenters (E) der Vorfermenter (A) automatisch mit frischer Maische nachgefüllt wird.

5. Verfahren nach den Ansprüchen 1 bis 4, dadurch gekennzeichnet, dass im Vorfermenter (A) bis zu einem Alkoholgehalt von 0,5 bis 3 Volumenprozent vorversäuert wird.

6. Verfahren nach den Ansprüchen 1 bis 5, dadurch gekennzeichnet, dass das Volumenverhältnis von Vorfermenter (A) zu Erntefermenter (E) 1,7 bis 3,5 beträgt.

## Claims

1. A method for the submersed, automatically controlled production of fermentation vinegar from ethanol in two steps, whereby product fermenters (E) are provided for one or more pre-fermenters (A) and the pre-fermenters (A) are partially run with fresh mash for the renewal of the biomass, wherein the product valve (11') and the product pump (9') of the product fermenter (E) are controlled by the rise in the amount of dissolved oxygen in the mash after the alcohol has been nearly completely consumed.

2. A method according to claim 1, wherein the product fermenter (E) is completely emptied out after pre-acidification up to the terminal concentration of ethanol attainable by biological oxidation with Acetobacter.

3. A method according to claims 1 and 2, wherein after completion of the emptying process the product fermenter (E) is automatically filled up from the pre-fermenter (A).

4. A method according to claims 1 to 3, wherein the pre-fermenter (A) is automatically replenished with fresh mash after the product fermenter (E) has been filled up.

5. A method according to claims 1 to 4, wherein the pre-fermenter (A) is pre-acidified up to an alcohol content of from 0.5 to 3 percent by volume.

6. A method according to claims 1 to 5, wherein the volume ratio of pre-fermenter (A) to product fermenter (E) is from 1.7 to 3.5.

## Revendications

1. Procédé pour la production en immersion, commandée automatiquement, de vinaigre de fermentation à partit de l'éthanol, en deux phases, un fermenteur de soutirage (E) et ces préfermenteurs (A) étant alimentés partiellement en moût frais pour renouveler la biomasse, caractérisé en ce que la vanne de soutirage (11') et la pompe (9') du fermenteur de soutirage (E) sont commandées par l'accroissement de l'oxygène dissous dans le substrat après épuisement pratiquement complet de l'alcool.

2. Procédé selon la revendication 1, caractérisé en ce que le fermenteur de soutirage (E) est complètement vidé après acification par les acétobacters jusqu'à la concentration finale en éthanol que permet d'obtenir l'oxydation biologique.

3. Procédé selon les revendications 1 et 2, caractérisé en ce que le fermenteur de soutirage (E) est automatiquement rempli à partir du préfermenteur (A) lorsque son soutirage est terminé.

4. Procédé selon les revendications 1 à 3, caractérisé en ce que le préfermenteur (A) est automatiquement achevé à l'aide de moût frais après le remplissage du fermenteur de soutirage (E).

5. Procédé selon les revendications 1 à 4, caractérisé en ce que la préacidification est conduite dans le préfermenteur (A) jusqu'à une teneur en alcool de 0,5 à 3 pourcent en volume.

6. Procédé selon les revendications 1 à 5, caractérisé en ce que le rapport du volume du préfermenteur (A) à celui du fermenteur de soutirage (E) est de 1,7 à 3,5.

0 026 527